# EUROPEAN PATENT SPECIFICATION

(11) **EP 0 758 542 B1**
(45) Date of publication and mention of the grant of the patent: **12.03.2003**
(21) Application number: 95305718.9
(22) Date of filing: 16.08.1995
(51) Int. Cl.: A61F 2/24

(54) **Heart valve prosthesis incorporating electronic monitoring and/or pacing circuitry**
Herzklappenprothese mit elektronischer Überwachungs- und/oder Schrittmacherschaltung
Prothèse de valve cardiaque munie d'un circuit électronique de surveillance et/ou de stimulation

(43) Date of publication of application: 19.02.1997
(73) Proprietor: Villafana, Manuel A., Minneapolis, Minnesota 55447 (US)
(72) Inventor: Villafana, Manuel A., Minneapolis, Minnesota 55447 (US)
(74) Representative: MacGregor, Gordon

(56) References cited:
- US-A- 4 661 107
- US-A- 4 769 032
- US-A- 4 979 955

## Description

### BACKGROUND OF THE INVENTION

I. Field of the Invention: This invention relates generally to an improved heart valve prosthesis, and more particularly to such a prosthesis device which may be implanted to replace a defective natural or artificial heart valve and which incorporates electronics for monitoring and telemetering operational conditions of the valve and other biodata to an external receiver, as well as for providing stimulation pulses for pacing. The improved prosthesis device of the present invention employs at least one, but preferably two or even three occluder means, each in the form of a flat or curved plate or leaflet, both functioning hemodynamically by a periodic opening and closing motion which is created through normal pumping action of the heart. The improved prosthesis device may alternatively employ multiple occluder means, such as two or three flat or curved plates, or alternatively, a ball and cage check occluder. A sensor is incorporated for detecting such things as the movement of the leaflets and a transmitter/receiver (transceiver) is incorporated for sending and receiving data and commands from an external apparatus.

I. Discussion of the Prior Art: As is well known in the art, prosthetic heart valves function essentially as check valves. Blood flow, which occurs as a result of the natural pumping action of the heart, causes periodic opening of the leaflets, with the system pressure closing the leaflets during periods of diastole when in the aortic position or during periods of systole when in the atrial-ventricular position.

A variety of prosthetic heart valves have been proposed and utilized in the past. Certain of these prosthetic devices have employed a caged ball arrangement which also function and control blood flow in response to normal pumping action of the heart. The caged-ball designs have been found objectionable from a psychologic standpoint because of the audible clicking sounds emitted as the ball is made to seat and unseat relative to the opening in the valve body. Other heart valve prostheses have employed occluders in the form of either a round disk or a pair of semi-circular and semi-elliptical plates. The latter are normally referred to as bi-leaflet valves. While various materials of construction have been employed in the past, the more recently utilized heart valve prostheses have been fabricated essentially from pyrolytic carbon.

Bi-leaflet valves normally employ a strategically designed pivot means to appropriately guide and otherwise control the motion of the leaflets as they are made to move between their open and closed dispositions. In addition, means have been provided to control or limit the extent of motion to which the leaflets are subjected during opening and closing, thereby providing an arrangement wherein the motion of the individual leaflets is carefully guided, controlled, limited and maintained.

It is further known that blood components, including those cells normally found in human blood, are extremely fragile and delicate. These cells can be damaged and/or destroyed if subjected to unusual mechanical forces. Thus, care must be taken to control the nature of the forces created during the occurrence of relative motion between the leaflets and their surrounding annular body. For example, reduction of the occurrences of rubbing contact between stationary and moving surfaces is of importance when such contact is likely to cause mechanical damage to the various cell types present in blood. The design and configuration of the heart valve prosthesis of the present invention is such that care has been taken to reduce the creation of zones or areas where blood passing through the device is exposed to substantial mechanical forces. As such, the operation of the valve of the present invention is practically noiseless and conventional acoustic techniques cannot be used to assess operational performance of such "silent" valves. Accordingly, it is desirable that some alternative method of monitoring valve performance and other physiologic parameters be incorporated in or with the valve.

US 4 979 955 discloses a power assisted mechanism that is applied to prosthetic valves as a way of initiating closure of the valve at the appropriate moment in the pump cycle to minimise traumatic hemolysis of red blood cells, and to provide a negative charge to the valve as a way of repelling red blood cells and platelets to reduce thrombogenic phenomena and to provide a cushioning force field effect to reduce red blood cell hemolysis associated with turbulent flow across the valve. In one embodiment, a known tilting disc valve comprising an annular frame or cage and a disc pivotably mounted thereon has a fixed electromagnet attached to the annular frame and a permanent magnet attached to the tilting disc. When energised, the resultant magnet field will repel the permanent magnet located on the free floating disc thereby closing the valve. An important feature involves determining the appropriate moment in the cardiac cycle to initiate active closure of the valve. In a method of triggering or initiating closure of the valve, the electrical activity of the heart is monitored and at the appropriate time in the cardiac cycle, as determined by the electrical wave form, active closure of the device may be initiated. This can be accomplished by using a monitoring and control circuit which is similar to known monitoring and control circuits associated with pacemaker and implantable cardioverter/defibrillator devices.

According to the present invention there is provided, in combination, an artificial heart valve of the type having a tubular body member, defining a lumen and pivotally supporting at least one occluder, said body member having a sewing cuff covering an exterior surface of said body member; and an electronic sensor module characterised in that the sensor module is disposed between said sewing cuff and said exterior surface, and in that said sensor module incorporates a sensor element for detecting movement of said at least one occluder.

### SUMMARY OF THE INVENTION

The heart valve prosthesis of the present invention comprises an improvement which is particularly adapted for use as a modification of the valve described in U.S. Patent No. 5,354,330 and entitled "HEART VALVE PROSTHESIS" It will be understood, of course, that the features of the present invention are readily adaptable for implementation in other types of valves, including other mechanical valves as well as tissue valves. In U. S. Patent No. 5,354,330 there is described a heart valve prosthesis having a generally annular body member with an interior surface defining a central passageway or lumen for blood flow therethrough. The annular body member is preferably formed from pyrolytic carbon and is provided with means for supporting a pair of pivotally moveable leaflets or occluders within the annular body in such a way that they alternately open and close under influence of the blood being pumped by the heart. The valves thereby allow only a unidirectional flow of blood through the lumen or passageway of the heart valve. The annular body is suspended within a stiffening ring and a locking wire of a predetermined diameter is contained within annular grooves formed in the outer surface of the annular body and the inner surface of the stiffening ring to prevent longitudinal movement of the annular body relative to the stiffening ring.

A fabric sewing cuff encompasses the outer surfaces of the stiffening ring. Disposed between a portion of the fabric sewing cuff and the stiffening ring is a moisture-proof, annular container for housing an electronic sensor, as well as a printed circuit board populated with appropriate electronic components for implementing a RF transceiver and an appropriate active or passive power supply for energizing same.

The sensor may comprise any one of a number of types capable of detecting the motion of the occluder and for providing an electrical signal indicative thereof to the transceiver circuitry. A blood flow sensor may also be used. The transceiver then functions to transmit analog or digital information to a suitable monitor located outside of the body of the patient in which the valve of the present invention is implanted. This sensor, in detecting the motion of the leaflets, may function to detect blood flow, cardiac output, as well as certain other information indicative of the function of the heart. The implanted transceiver can also receive commands from an external transmitter to accomplish some physiologic function, such as providing cardiac stimulating pulses in the event that post-surgical heart block occurs, or alternatively to control other cardiac arrhythmias.

### DESCRIPTION OF THE DRAWINGS

The foregoing features, objects and advantages of the invention will become apparent to those skilled in the art from the following detailed description of the preferred embodiment, especially when considered in conjunction with the accompanying drawings in which like numerals in the several views refer to corresponding parts.
Figure 1 illustrates a cross-section through the heart illustrating heart valve prostheses in accordance with the present invention used as replacements for the mitral valve and an aortic valve;
Figure 2 is a greatly enlarged partial cross-sectional longitudinal view showing an electronics module contained within the sewing cuff of a typical heart valve prosthesis for transmitting and receiving information and commands transcutaneously to an external monitor system; and
Figure 3 is a block diagram of the electronics associated with the heart valve prosthesis in accordance with the present invention.

### DESCRIPTION OF THE PREFERRED EMBODIMENT

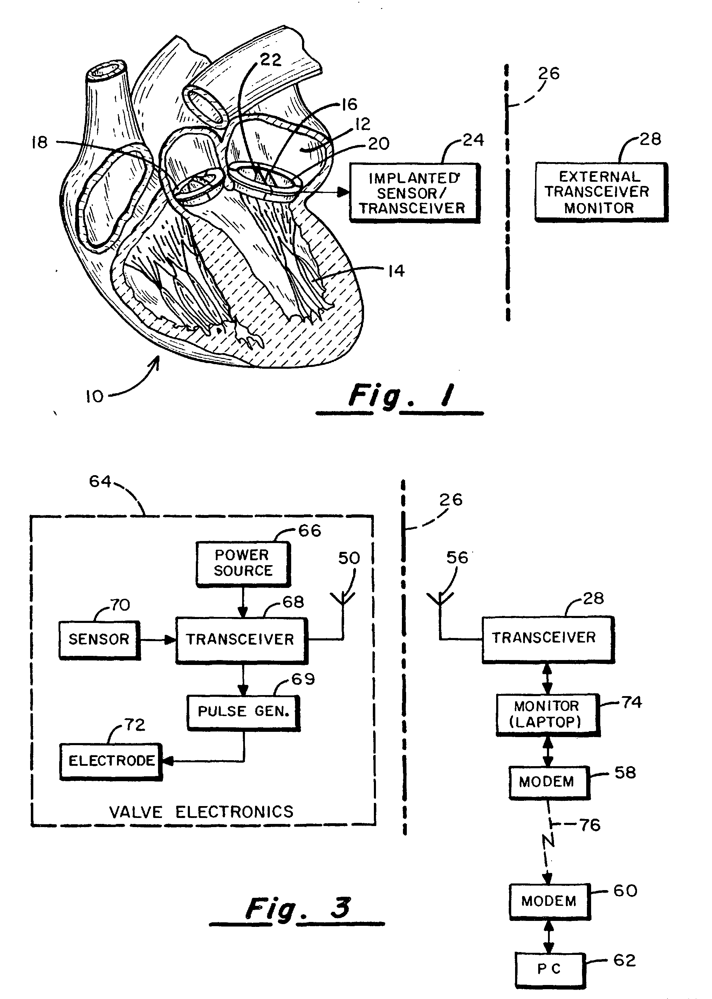

For purposes of reference only, there is shown in Figure 1 a vertically sectioned human heart 10 showing the right atrium 12, the left ventricle 14 and an artificial heart valve prosthesis 16 replacing the normal mitral valve and a further prosthetic heart valve 18 replacing the subject's aortic valve. The present invention may also be incorporated in a prosthesis replacing the tricuspid valve.

As will be explained in greater detail hereinbelow, either or both of the heart valve prostheses 16 and 18 may incorporate therein an appropriate sensor positioned and configured to detect the normal movement of the leaflets 20 and 22 as they open and close under influence of the blood flowing through the annular body of the artificial heart valve 16. The sensor module 24 is preferably contained within the valve structure, as will be further described, but in accordance with another feature of the present invention, the transceiver electronics may be in a separate moisture-proof container and coupled to the heart valve 16 in such a way that it can receive, amplify and transmit information transcutaneously through the chest wall 26 to an external transceiver/monitor 28.

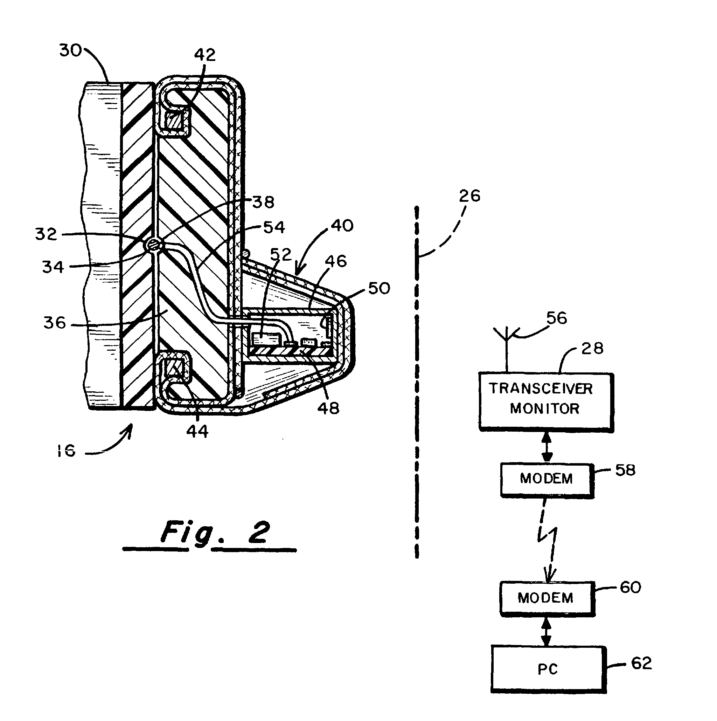

Referring next to Figure 2, the heart valve 16 is seen to include an annular body member 30 preferably formed from, but not limited to, pyrolytic carbon in which the occluder or leaflets 20 and 22 are pivotally suspended. The view of Figure 2 does not show the leaflets themselves. As is fully explained in US-A-5,354,330 the outer peripheral surface of the annular body 30 includes a groove 32 for receiving a lock wire therein. Surrounding the valve body 30 is a metal or plastic stiffening ring 36 which also has an annular groove 38 formed therein for receiving a portion of the lock wire 34 therein. Stiffening ring 36 may be constructed of a non-conductive material. The tolerances are such that the lock wire 34 prevents relative longitudinal sliding motion between the annular body 30 and its surrounding stiffening ring 36.

Also formed inwardly of the annular surface of the stiffening ring 36 are upper and lower grooves designed to contain and trap a fabric sewing cuff 40 are first and second lock rings 42 and 44. Because of the manner in which the fabric sewing cuff 40 is wrapped about the stiffening ring 36 and the lock rings 42 and 44, the sewing cuff is prevented from coming free of the assembled heart valve 16.

In the embodiment of the heart valve disclosed in US-A-5,354,330 a plastic filler ring, identified therein by numeral 58, is fitted between the exterior of the stiffening ring 36 and the sewing cuff to create an annular flange. In the embodiment shown in Figure 2 hereof, the filler ring is replaced by a moisture-proof ring-shaped container or housing 46, which is preferably a welded titanium can designed to house the circuitry identified by numeral 24 in Figure 1. The cross-sectional view of Figure 2 shows within can 46 a printed circuit substrate 48 which is populated with integrated and discrete electronic components comprising a transceiver circuit. A printed circuit coil antenna 50 is coupled to the electronic circuitry on the printed circuit board 48 for transmitting and receiving electrical signals.

Also housed within the container 46 is a suitable sensor element 52. The sensor 52 may be capacitive or inductive in nature and appropriately arranged to sense the movement of the leaflets 20 and 22 (Figure 1). An inductive transducer may comprise a coil positioned in the stiffening ring to be cut by magnetic flux from a dipole mounted on or in the occluder. Those skilled in the art can appreciate that other forms of transducers for detecting movement and/or fluid flow through the annular body 30 can also be utilized in carrying out the principles of the present invention. For example, an ultrasonic Doppler flow sensor may be used to detect the behavior of the blood flow through the valve body as the leaflets open and close. Also, a quartz crystal transducer can be employed to sense vibration.

A suitable active power source, such as a lithium iodide battery of the type used in cardiac pacemakers may also be contained within the housing 46. Alternatively, a passive power source comprising the antenna 50 coupled through a suitable diode rectifying circuit to an energy storing capacitor may be provided so that the electronics within the housing 46 can be powered by a continuous wave RF signal transmitted percutaneous from the transceiver/monitor circuit 28 to the implanted valve electronics percutaneously.

When it is considered that the AV node, which controls the rate of the ventricles, is located in close proximity to both the mitral valve and the aortic valve and given the fact that during the post-operative period it is not uncommon for a patient to go into temporary or permanent heart block, it is also desirable that a means be provided for pacing the heart. In the past, it has been the practice to install temporary pacing leads percutaneously for connection to an external pacemaker. The heart pacing leads are relatively costly and require a channel through the skin which can become a site for infection, requiring ultimate removal of the leads.

Because the lock wire 32 is a conductor and is in blood contact, by connecting that wire to an electronic pulse generator within the housing 46 and controlling the pulse generator through an RF link between the antenna 56 of transceiver 28 and the antenna 50 of the implanted transceiver, pacing of the ventricles can be achieved.

In monitoring the operation of the implanted heart valve, information telemetered from the implanted electronics module to the external transceiver/monitor 28 allows assessment of such things as leaflet movement, pannus overgrowth on the ring, cardiac arrhythmias, cardiac output.

If it is desired to be able to assess a patient at a distance, the transceiver 28 located proximate the patient may be coupled, via modems 58 and 60, to a remotely located personal computer 62 located, for example, in the office of the cardiac surgeon, cardiologist, or paramedical personnel.

Figure 3 is a system block diagram showing the implanted electronics within the broken line box 64. As earlier mentioned, the implanted electronic components may be contained as an integral part of the heart valve in the manner illustrated in Figure 2 or, alternatively, the power source 66 and transceiver 68 may be separately packaged within a moisture-proof housing external to the heart but still within the patient's chest area. Only the sensor 70 and the electrode 72 would be disposed on or in the prosthetic heart valve itself. As before, messages may be transmitted percutaneously through the chest wall 26 to a local transceiver 28 and monitor 74 which may be a lap-top computer or similar device programmed to analyze the information provided by the sensor 70 to the implanted transceiver 68 and transmitted thereby to the transceiver 28. By using modems 58 and 60 joined to a telephone link 76, it is possible to monitor/control operation of the system from a remote location.

In the event an arrhythmia is detected, the system can be programmed to automatically initiate cardiac pacing. The monitor 74 will provide command pulses at a predetermined pacing rate to the transceiver 28 which will then send a command to the implanted transceiver 68 so that it can control the application of cardiac stimulating pulses from the pulse generator 69 to the electrode 72. As mentioned in connection with Figure 2, the electrode 72 may comprise the lock wire 34 forming a part of the heart valve itself.

This invention has been described herein in considerable detail in order to comply with the Patent Statutes and to provide those skilled in the art with the information needed to apply the novel principles and to construct and use such specialized components as are required. However, it is to be understood that the invention can be carried out by specifically different equipment and devices, and that various modifications, both as to the equipment details and operating procedures, can be accomplished without departing from the scope of the invention itself.

For example, rather than disposing the electronics module in a moisture-proof container located between the sewing cuff 40 and the stiffening ring 36, some or all of the components comprising the electronics module may also be embedded in the stiffening ring 36. Hence the scope of the invention is to be determined from the following claims, when properly interpreted in light of the prior art.

## Claims

1. In combination, an artificial heart valve (16) of the type having a tubular body member (30), defining a lumen and pivotally supporting at least one occluder (20:22), said body member having a sewing cuff (40) covering an exterior surface of said body member; and an electronic sensor module (24) **characterised in that** the sensor module is disposed between said sewing cuff and said exterior surface, and **in that** said sensor module (24) incorporates a sensor element (52) for detecting movement of said at least one occluder.

2. The combination of Claim 1 wherein the sensor element detects movement of said at least one occluder between an open and a closed disposition relative to said lumen.

3. The combination as in Claim 1 or 2 wherein said sensor module (24) further includes a signal transceiver (28) coupled to said sensor element (52), and means for energizing said signal transceiver.

4. The combination as claimed in claim 1 wherein the sensor element (52) detects movement of said at least one occluder (20:22) relative to said body member and produces a first electrical signal indicative of such movement, and a tranceiver means (28) and a source of electrical energy coupled thereto disposed within a moisture impervious housing (46) and with said transceiver means being operatively coupled to said sensing means for receiving said first electrical signal and for producing a second electrical signal capable of percutaneous electronic transmission of information related to said first electrical signal from said housing to a location external to a patient in which the artificial heart valve is implanted, the combination further comprising monitoring means (62) external to the body of the patient for receiving and analysing further information.

5. The apparatus as in Claim 4 wherein said sensing means (24) senses capacitive changes resulting from movement of said occluder.

6. The apparatus as in Claim 4 wherein said sensing means (24) senses inductive changes resulting from movement of said occluder.

7. The apparatus as in Claim 4 wherein said sensing means (24) senses vibration of said body member resulting from movement of said occluder.

8. The apparatus as in Claim 4 wherein said sensing means (24) is not disposed in a blood path passing through said body member.

9. The combination as claimed in Claim 3 wherein the sensor module (24) includes means (46) for encapsulating said sensor element (52), signal transceiver (28) and energising means in a moisture impervious container.

10. The combination as claimed in Claim 2 further including an implantable transceiver module (28), said transceiver module comprising a radio-telemetry circuit operatively coupled to said sensor element (52) for receiving an input signal therefrom relating to the detected movement of said at least one occluder and for providing an output signal to an antenna (50).

11. The combination as in Claim 10 wherein said transceiver module (28) further includes means for energizing said radio-telemetry circuit and said sensor element (52).

12. The combination as in Claim 11 wherein said sensor element (52) comprises a capacitance element.

13. The combination as in Claim 11 wherein said sensor element (52) comprises an inductance element.

14. The combination as in Claim 11 wherein said sensor element (52) comprises a vibration sensor.

15. The combination as in Claim 14 wherein said vibration sensor (52) is a quartz crystal.

16. The combination as in Claim 10 wherein said implantable transceiver module (68) is external to said artificial heart valve.

17. The combination as in Claim 10 and further including electrode means (72) incorporated into said artificial heart valve and coupled to said transceiver module (28) for stimulating heart tissue upon receipt of heart pacing signals from an exterior transceiver (28) via said antenna (50).

18. The combination as claimed in Claim 1 further comprising an electrode (72) disposed on said tubular body member; a transceiver module (28) including a moisture impervious housing (16), said housing containing an electronic transceiver (28) having an output coupled to said electrode, and means (58, 60, 76) located remote from said electronic transceiver for transmitting pulses for pacing cardiac tissue to said electronic transceiver.

19. The combination as in Claim 18 wherein said means for transmitting pulses comprises a radio-telemetry device.

20. The combination as in Claim 18 wherein said moisture impervious housing (46) is disposed between said sewing cuff (40) and said body member (30).

21. The combination as in Claim 19 wherein said radio-telemetry device is external to the body in which said heart valve is implanted.

## Patentansprüche

1. Kombination aus einer künstlichen Herzklappe (16) von der Art, die ein rohrförmiges Körperelement (30) aufweist, welches einen Hohlraum definiert und an dem zumindest eine Schließeinrichtung (20, 22) drehbar angebracht ist, wobei das Körperelement eine Nähmanschette (40) aufweist, die eine Außenfläche des Körperelements abdeckt, und einem elektronischen Sensormodul (24), **dadurch gekennzeichnet, dass** das Sensormodul zwischen der Nähmanschette und der Außenfläche angeordnet ist und dadurch, dass das Sensormodul (24) ein Sensorelement (52) zum Ermitteln der Bewegung der zumindest einen Schließeinrichtung umfasst.

2. Kombination nach Anspruch 1, wobei das Sensorelement die Bewegung der zumindest einen Schließeinrichtung zwischen einer offenen und einer geschlossenen Anordnung relativ zum Hohlraum ermittelt.

3. Kombination nach Anspruch 1 oder 2, wobei das Sensormodul (24) ferner einen mit dem Sensorelement (52) verbundenen Signal-Sender/Empfänger (28) und Einrichtungen zum Versorgen des Signal-Sender/Empfängers mit Energie umfasst.

4. Kombination nach Anspruch 1, wobei das Sensorelement (52) die Bewegung der zumindest einen Schließeinrichtung (20, 22) relativ zum Körperelement ermittelt und ein erstes elektrisches Signal erzeugt, das eine solche Bewegung indiziert, und eine Sender/Empfänger-Einrichtung (28) und eine damit verbundene elektrische Energiequelle in einem feuchtigkeitsdichten Gehäuse (46) angeordnet sind, und wobei die Sender/Empfänger-Einrichtung betriebsfähig mit der Sensoreinrichtung verbunden ist, um das erste elektrische Signal zu empfangen und ein zweites elektrisches Signal zu erzeugen, welches Informationen bezüglich des ersten elektrischen Signals von dem Gehäuse zu einer Stelle außerhalb eines Patienten, in den die künstliche Herzklappe implantiert ist, in perkutaner elektronischer Weise übertragen kann, wobei die Kombination ferner Überwachungseinrichtungen (62) außerhalb des Körpers des Patienten zum Empfangen und Analysieren weiterer Informationen umfasst.

5. Vorrichtung nach Anspruch 4, wobei die Sensoreinrichtung (24) kapazitive Veränderungen erfasst, die aus der Bewegung der Schließeinrichtung resultieren.

6. Vorrichtung nach Anspruch 4, wobei die Sensoreinrichtung (24) induktive Veränderungen erfasst, die aus der Bewegung der Schließeinrichtung resultieren.

7. Vorrichtung nach Anspruch 4, wobei die Sensoreinrichtung (24) Vibrationen des Körperelements erfasst, die aus der Bewegung der Schließeinrichtung resultieren.

8. Vorrichtung nach Anspruch 4, wobei die Sensoreinrichtung (24) nicht in einer Blutbahn angeordnet ist, die durch das Körperelement verläuft.

9. Kombination nach Anspruch 3, wobei das Sensormodul (24) Einrichtungen (46) zum Verkapseln des Sensorelements (52), des Signal-Sender/Empfängers (28) und der Energieversorgungseinrichtung in einem feuchtigkeitsdichten Behälter umfasst.

10. Kombination nach Anspruch 2, die ferner ein implantierbares Sender/Empfänger-Modul (28) umfasst, wobei das Sender/Empfänger-Modul eine Radiotelemetrieschaltung umfasst, die betriebsfähig mit dem Sensorelement (52) verbunden ist, um von diesem ein Eingangssignals zu empfangen, welches sich auf die ermittelte Bewegung der zumindest einen Schließeinrichtung bezieht, und ein Ausgangssignal an eine Antenne (50) abzugeben.

11. Kombination nach Anspruch 10, wobei das Sender/Empfänger-Modul (28) ferner Einrichtungen zum Versorgen der Radiotelemetrieschaltung und des Sensorelements (52) mit Energie umfasst.

12. Kombination nach Anspruch 11, wobei das Sensorelement (52) ein Kapazitätselement umfasst.

13. Kombination nach Anspruch 11, wobei das Sensorelement (52) ein Induktivitätselement umfasst.

14. Kombination nach Anspruch 11, wobei das Sensorelement (52) einen Vibrationssensor umfasst.

15. Kombination nach Anspruch 14, wobei der Vibrationssensor (52) ein Quarzkristall ist.

16. Kombination nach Anspruch 10, wobei sich das implantierbare Sender/Empfänger-Modul (68) außerhalb der künstlichen Herzklappe befindet.

17. Kombination nach Anspruch 10, die ferner Elektrodeneinrichtungen (72) umfasst, die in der künstlichen Herzklappe enthalten und mit dem Sender/Empfänger-Modul (28) verbunden sind, um das Herzgewebe beim Empfang von Herzschrittmachersignalen von einem externen Sender/Empfänger (28) über die Antenne (50) zu stimulieren.

18. Kombination nach Anspruch 1, die ferner eine Elektrode (72), welche an dem rohrförmigen Körperelement angeordnet ist, ein Sender/Empfänger-Modul (28), das ein feuchtigkeitsdichtes Gehäuse (16) umfasst, wobei das Gehäuse einen elektronischen Sender/Empfänger (28) mit einem mit der Elektrode verbundenen Ausgang enthält, und Einrichtungen (58, 60, 76) umfasst, die entfernt von dem elektronischen Sender/Empfänger angeordnet sind, um Impulse zum Anpassen des Tempos des Herzgewebes an den elektronischen Sender/Empfänger zu übertragen.

19. Kombination nach Anspruch 18, wobei die Einrichtung zum Übertragen von Impulsen eine Radiotelemetrieeinrichtung umfasst.

20. Kombination nach Anspruch 18, wobei das feuchtigkeitsdichte Gehäuse (46) zwischen der Nähmanschette (40) und dem Körperelement (30) angeordnet ist.

21. Kombination nach Anspruch 19, wobei sich die Radiotelemetrieeinrichtung außerhalb des Körpers befindet, in den die Herzklappe implantiert ist.

## Revendications

1. Combinaison d'une valvule cardiaque artificielle (16) du type comprenant un élément de corps tubulaire (30), définissant une lumière et supportant de manière pivotante au moins un obturateur (20:22), ledit élément de corps ayant une calotte à couture (40) recouvrant une surface extérieure dudit élément de corps ; et d'un module à capteur électronique (24), **caractérisée en ce que** le module à capteur est disposé entre ladite calotte à couture et ladite surface extérieure, et **en ce que** ledit module à capteur (24) incorpore un élément de capteur (52) destiné à détecter le déplacement dudit au moins un obturateur.

2. Combinaison selon la revendication 1, dans laquelle l'élément de capteur détecte le déplacement dudit au moins un obturateur entre une disposition ouverte et une disposition fermée par rapport à ladite lumière.

3. Combinaison selon la revendication 1 ou 2, dans laquelle ledit module à capteur (24) inclut, en outre, un émetteur-récepteur de signal (28) couplé audit élément de capteur (52), et des moyens destinés à activer ledit émetteur-récepteur de signal.

4. Combinaison selon la revendication 1, dans laquelle l'élément de capteur (52) détecte le déplacement dudit au moins un obturateur (20:22) par rapport audit élément de corps et produit un premier signal électrique indicatif d'un tel déplacement, et un moyen à émetteur-récepteur (28) et une source d'énergie électrique qui lui est couplée étant disposés au sein d'un logement étanche à l'humidité (46), ledit moyen à émetteur-récepteur étant couplé, en fonctionnement, audit moyen de détection pour recevoir ledit premier signal électrique et pour produire un second signal électrique permettant la transmission électronique percutanée de l'information associée audit premier signal électrique provenant dudit logement jusqu'à un emplacement extérieur à un patient dans lequel est implantée la valvule cardiaque artificielle, la combinaison comprenant en outre des moyens de contrôle (62), extérieurs au corps du patient, destinés à recevoir et analyser des informations supplémentaires.

5. Dispositif selon la revendication 4, dans lequel ledit moyen de détection (24) détecte les variations capacitives résultant du déplacement dudit obturateur.

6. Dispositif selon la revendication 4, dans lequel ledit moyen de détection (24) détecte les variations inductives résultant du déplacement dudit obturateur.

7. Dispositif selon la revendication 4, dans lequel ledit moyen de détection (24) détecte les vibrations dudit élément de corps résultant du déplacement dudit obturateur.

8. Dispositif selon la revendication 4, dans lequel ledit moyen de détection (24) n'est pas disposé sur un trajet du sang passant par ledit élément de corps.

9. Combinaison selon la revendication 3, dans laquelle le module à capteur (24) inclut des moyens (46) destinés à encapsuler ledit élément de capteur (52), ledit émetteur-récepteur de signal (28) et lesdits moyens d'activation dans un conteneur étanche à l'humidité.

10. Combinaison selon la revendication 2, incluant en outre un module à émetteur-récepteur implantable (28), ledit module à émetteur-récepteur comprenant un circuit de radiotélémétrie couplé en fonctionnement audit élément de capteur (52) pour recevoir, de celui-ci, un signal d'entrée se rapportant au déplacement détecté dudit au moins un obturateur et pour transmettre un signal de sortie à une antenne (50).

11. Combinaison selon la revendication 10, dans laquelle ledit module à émetteur-récepteur (28) inclut en outre des moyens destinés à activer ledit circuit de radiotélémétrie et ledit élément de capteur (52).

12. Combinaison selon la revendication 11, dans laquelle ledit élément de capteur (52) comprend un élément de capacité.

13. Combinaison selon la revendication 11, dans laquelle ledit élément de capteur (52) comprend un élément d'inductance.

14. Combinaison selon la revendication 11, dans laquelle ledit élément de capteur (52) comprend un capteur de vibrations.

15. Combinaison selon la revendication 14, dans laquelle ledit capteur de vibrations (52) est un cristal de quartz.

16. Combinaison selon la revendication 10, dans laquelle ledit module à émetteur-récepteur implantable (68) est extérieur à ladite valvule cardiaque artificielle.

17. Combinaison selon la revendication 10, et incluant en outre des moyens à électrode (72) incorporés dans ladite valvule cardiaque artificielle et couplés audit module à émetteur-récepteur (28) pour stimuler le tissu cardiaque lors de la réception de signaux de stimulation cardiaque provenant d'un émetteur-récepteur extérieur (28) via ladite antenne (50).

18. Combinaison selon la revendication 1, comprenant en outre une électrode (72) disposée sur ledit élément de corps tubulaire ; un module à émetteur-récepteur (28) incluant un logement étanche à l'humidité (16), ledit logement contenant un émetteur-récepteur électronique (28) ayant une sortie couplée à ladite électrode, et des moyens (58, 60, 76) situés à distance dudit émetteur-récepteur électronique pour transmettre audit émetteur-récepteur électronique des impulsions de stimulation du tissu cardiaque.

19. Combinaison selon la revendication 18, dans laquelle lesdits moyens pour transmettre des impulsions comprennent un dispositif de radiotélémétrie.

20. Combinaison selon la revendication 18, dans laquelle ledit logement étanche à l'humidité (46) est disposé entre ladite calotte à couture (40) et ledit élément de corps (30).

21. Combinaison selon la revendication 19, dans laquelle ledit dispositif de radiotélémétrie est extérieur au corps dans lequel est implantée ladite valvule cardiaque.
